# EUROPÄISCHE PATENTANMELDUNG

(11) **EP 0 945 108 A2**
(43) Veröffentlichungstag der Anmeldung: **29.09.1999**
(21) Anmeldenummer: 99104191.4
(22) Anmeldetag: 02.03.1999
(51) Int. Cl.: A61F 2/30, A61F 2/36

(54) **Endoprothese mit einem Verankerungsschaft**

(30) Priorität: 24.03.1998 DE 29805344 U
(71) Anmelder: orto MAQUET GmbH & Co. KG, 76437 Rastatt (DE)
(72) Erfinder: Gerhardt, Dr.Harald, 76131 Karlsruhe (DE)
(74) Vertreter: Schaumburg, Thoenes & Thurn

(57) **Zusammenfassung**

Eine Endoprothese mit einem Verankerungsschaft (12), der zum Einsetzen in eine in einem Röhrenknochen (20) erzeugte Aussparung (18) bestimmt ist und auf mindestens einem Teil seiner Länge einen mindestens annähernd polygonalen Querschnitt hat, ist so ausgebildet, daß sich die Querschnittskonturlinie des Schaftes (12) aus geraden und gekrümmten Linienabschnitten (22, 24, 26, 28 bzw. 38, 40, 42, 44, 60, 62) zusammensetzt und daß der minimale Krümmungsradius (54) der gekrümmten Linienabschnitte (38, 40, 42, 44, 60, 62) stets größer oder gleich dem Radius eines für die Herstellung der Aussparung (18) im Knochen (20) verwendeten Fräskopfes ist.

## Beschreibung

Die Erfindung betrifft eine Endoprothese mit einem Verankerungsschaft, der zum Einsetzen in eine in einem Rohrenknochen erzeugte Aussparung bestimmt ist, wobei der Verankerungsschaft auf mindestens einem Teil seiner Länge einen mindestens annähernd polygonalen Querschnitt hat.

Ein Beispiel für eine derartige Endoprothese ist der Teil eines künstlichen Hüftgelenkes, der in den Oberschenkelknochen eingesetzt wird und den Oberschenkelkopf und den Oberschenkelhals ersetzen soll. Um einen festen Sitz der Prothese in dem Knochen zu erreichen, muß die Aussparung zur Aufnahme des Schaftes entsprechend der Verankerungsphilosophie ("formfit" oder "pressfit") genau gearbeitet werden. In der Preis ist es nicht möglich, die Aussparung in dem Knochen so exakt herzustellen, daß der gewünschte Sitz entlang der gesamten Umfangsflache des Schaftes erreicht wird.

Daher wird derzeit bereits ein neues Operationsverfahren in der Preis angewandt, bei dem die Aussparung mit Hilfe eines programmgesteuerten Roboters ausgefräst wird. Dies ermöglicht eine hohe Genauigkeit beim Herstellen der Aussparung.

Bei der Verwendung der heute üblichen Geradschaftprothesen, die zum Erreichen einer Rotationsstabilität häufig einen rechteckigen Querschnitt haben, tritt jedoch das Problem auf, daß mit einem rotierenden Fräskopf keine Ecken gefräst werden können. Dies liegt vor allem daran, daß ein bestimmter Minimalradius des Fräskopfes aus Stabilitätsgründen nicht unterschritten werden kann. Der Fräskopf befindet sich an einem relativ langen Schaft und sein Durchmesser muß größer als der Schaftdurchmesser sein, damit der Schaft dem Fräskopf in die Aussparung folgen kann. Ist der Schaftdurchmesser zu gering, verbiegt sich der Schaft bei seitlichen Kräften, so daß die gewünschte Genauigkeit nicht erreicht werden kann. In der Regel beträgt daher der minimale Fräskopfradius ca. 5 mm.

Läßt man den Knochen in den Ecken entsprechend dem Fräskopfradius stehen, so besteht die Gefahr, daß der Knochen beim Einschlagen der Prothese mit ihren scharfen Rechteckkanten gespalten wird. Fräst man dagegen die Ecken vollständig aus, so entstehen beiderseits der Ecklinie Hohlräume, in denen der Prothesenschaft nicht am Knochen anliegt und in denen somit nicht nur die Prothesen keinen Halt findet, sondern auch die Gefahr besteht, daß sich der Knochen zurückbildet, da er dort nicht belastet wird.

Der Erfindung liegt die Aufgabe zugrunde, eine Endoprothese der eingangs genannten Art so auszubilden, daß der Schaft auf zumindest einem Teil seiner Länge mit seiner gesamten Umfangsfläche an der Aussparungswand anliegen kann.

Diese Aufgabe wird erfindungsgemäß dadurch gelöst, daß sich die Querschnittskonturlinie des Schaftes aus geraden und gekrümmten Linienabschnitten zusammensetzt und daß der minimale Krümmungsradius der gekrümmten Linienabschnitte stets größer oder gleich dem Radius eines für die Herstellung der Aussparung im Knochen verwendeten Fräskopfes ist. Damit entfällt die Gefahr, daß in den Eckbereichen zwischen geraden Linienabschnitten entweder Hohlräume entstehen oder Spaltkräfte von scharfen Kanten des Prothesenschaftes auf den Knochen ausgeübt werden.

Bei der bevorzugten Ausführungsform ist der Querschnitt des Schaftes auf zumindest einem Teil von dessen Länge im wesentlichen rechteckig, wobei die Krümmungsmittelpunkte der gekrümmten Linienabschnitte in den Eckbereichen auf der jeweiligen Winkelhalbierenden zweier aneinandergrenzender Seitenlinien liegt.

Der gekrümmte Linienabschnitt muß dabei nicht tangential in die jeweiligen geraden Linienabschnitte oder Seitenflächen des Schaftes übergehen. Vielmehr kann der jeweilige Krümmungsmittelpunkt auf der Winkelhalbierenden auch so verschoben werden, daß die entsprechende gekrümmte Linie durch den Schnittpunkt der beiden aneinandergrenzenden geraden Seitenlinien oder sogar außerhalb desselben verläuft.

Ein gekrümmter Linienabschnitt kann auch aus mehreren Teilabschnitten mit unterschiedlichen Krümmungsradien bestehen. Ferner kann ein gekrümmter Linienabschnitt aus gekrümmten Teilabschnitten bestehen, deren Krümmungsmittelpunkte einen Abstand voneinander haben.

Ein weitere Gestaltungsmöglichkeit ergibt sich dadurch, daß die Abstände des Krümmungsmittelpunktes eines gekrümmten Linienabschnittes von den durch diesen zu verbindenden Seitenlinien verschieden sind, d.h. daß der jeweilige Krümmungsmittelpunkt nicht auf der Winkelhalbierenden zwischen den beiden aneinandergrenzenden Seitenlinien liegt.

Grundsätzlich wird der Halt des Schaftes in dem Knochen verbessert, je größer die Kontaktfläche zwischen dem Schaft und dem Knochen ist. Diese Kontaktoberfläche kann dadurch vergrößert werden, daß - im Querschnitt betrachtet - im Bereich mindestens einer Seitenlinie ein zusätzlicher gekrümmter Linienabschnitt vorgesehen ist, dessen Krümmungsradius nicht kleiner als der minimale Krümmungsradius ist.

Weitere Merkmale und Vorteile der Erfindung ergeben sich aus der folgenden Beschreibung, welche in Verbindung mit den beigefügten Zeichnungen die Erfindung an Hand von Ausführungsbeispielen erläutert. Es zeigen:
- Figur 1: eine schematische Seitenansicht eines in einem Oberschenkelknochen zu verankernden Teiles eines künstlichen Hüftgelenkes,
- Figur 2: eine schematische Draufsicht auf den in Figur 1 dargestellten Prothesenteil und
- Figuren 3 bis 5: idealisierte Querschnitte durch verschiedene Ausführungsformen des Schaftes des in den Figuren 1 und 2 dargestellten Prothesenteiles ohne Berücksichtigung des künstlichen Schenkelhalses.

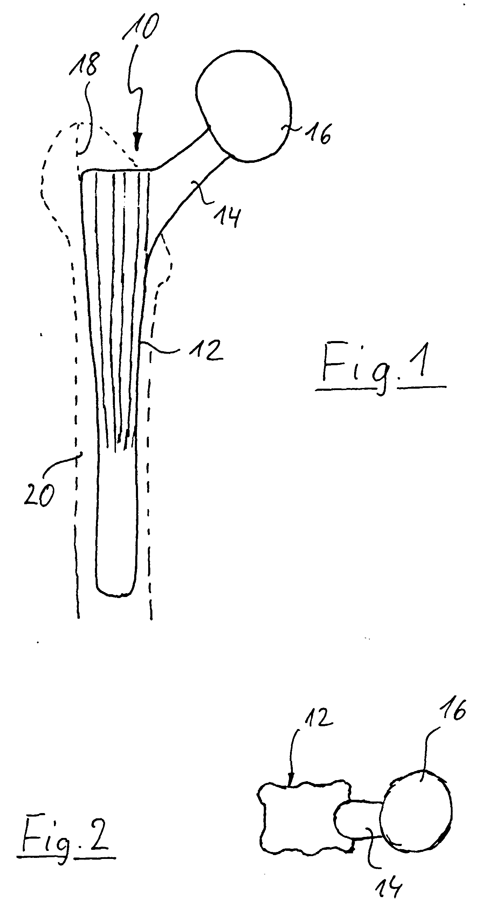

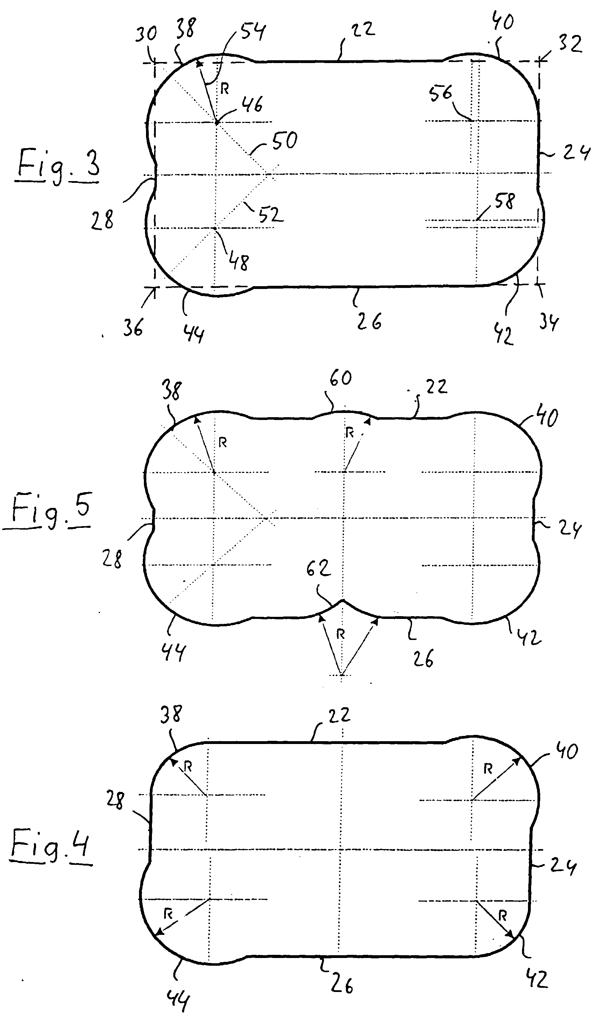

Die in Figur 1 dargestellte Endoprothese 10 ist Teil eines künstlichen Hüftgelenkes und umfaßt einen Schaft 12, der an seinem oberen Ende mit einem seitlich abstehenden, den Schenkelhals ersetzenden Fortsatz 14 verbunden ist, der eine den Oberschenkelkopf ersetzende Gelenkkugel 16 tragt. Der Schaft 12 ist in einer Aussparung 18 eingesetzt, die in einem gestrichelt angedeuteten Oberschenkelknochen 20 ausgefräst wurde. Der Schaft 12 besitzt zumindest in seinem oberen, dem Fortsatz 14 nahen Bereich einen im wesentlichen rechteckigen Querschnitt (Figur 2) und verjüngt sich zu seinem unteren Ende hin. Die Querschnittsform im oberen Schaftabschnitt, an dem die wesentlichen Kräfte angreifen und der für den Sitz der Prothese 10 im Knochen 20 besonders wichtig ist, soll nun an Hand der Figuren 3 bis 5 näher erläutert werden.

Der in Figur 3 dargestellte Schaftquerschnitt ist im wesentlichen rechteckig mit den Rechteckseiten oder geraden Linienabschnitten 22, 24, 26 und 28, wobei die ausgezogenen Teile dieser geraden Linien die tatsächliche Kontur wiedergeben, während die gestrichelten Abschnitte das Rechteck bis zu den gedachten Ecken 30, 32, 34 und 36 vervollständigen. In den Eckbereichen sind die geraden Linienabschnitte 22 bis 28 durch gekrümmte Linienabschnitte 38, 40, 42 und 44 miteinander verbunden.

Die beiden gekrümmten Linienabschnitte 38 und 44 sind jeweils von einem Kreisbogen um einen Mittelpunkt 46 bzw. 48 gebildet. Die Mittelpunkte 46, 48 liegen jeweils auf einer Winkelhalbierenden 50 bzw. 52 zwischen den aneinander angrenzenden Rechteckseiten 22 und 28 einerseits bzw. 26 und 28 andererseits. Der Radius 54 dieser Kreisbogen ist größer als der jeweilige Abstand des Mittelpunktes 46 bzw. 48 von den Rechteckseiten 22, 28 bzw. 26, 28. Dadurch gehen die Kreisbogen 38 und 44 nicht tangential in die jeweiligen Seitenlinien 22 und 28 bzw. 26 und 28 über, sondern bilden gleichsam Eckwülste an dem Schaft.

Auch die gekrümmten Abschnitte 40 und 42 an den Ecken 32 bzw. 34 sind von Kreisbogen gebildet, deren Kreismittelpunkt 56 bzw. 58 jedoch nicht auf der Winkelhalbierenden zwischen den Seiten 22 und 24 bzw. 24 und 26 liegt, sondern gegenüber dieser seitlich versetzt ist, so daß der Kreisbogen 40 tangential in die Seite 24 und der Kreisbogen 42 tangential in die Seite 26 übergeht, wie dies in Figur 3 zu erkennen ist, während die Kreisbögen 40, 42 über die jeweils andere Seite 22 bzw. 24 wulstförmig überstehen.

Die Eckbereiche des Schaftes können, wie in Figur 3 dargestellt, unterschiedlich oder aber auch gleich ausgebildet sein.

Bei der Ausführungsform gemäß Figur 4 sind einander entsprechende Teile wieder mit denselben Bezugszeichen wie in Figur 3 versehen. Bei der Ausführungsform gemäß Figur 4 haben die gekrümmten Linien- oder Konturabschnitte 38 und 42 einen kleineren Krümmungsradius als die gekrümmten Linienabschnitte 40, 44. Der Krümmungsradius braucht also nicht in allen Eckbereichen gleich zu sein. Er darf nur nicht kleiner als der minimal mögliche Radius des für die Aushöhlung des Knochens verwendeten Fräsers sein.

Das Ausführungsbeispiel gemäß Figur 5, bei dem ebenfalls entsprechende Teile wieder mit den gleichen Bezugszeichen wie in Figur 3 bezeichnet sind, unterscheidet sich von dem in Figur 3 dargestellten Ausführungsbeispiel vor allem dadurch, daß die gekrümmten Konturabschnitte in den Eckbereichen 30, 32, 34 und 36 aus Abschnitten mit unterschiedlichem Krümmungsradius zusammengesetzt sind und daß innerhalb der Längsseiten 22 und 24 der Querschnittskontur weitere gekrümmte Abschnitte 60 bzw. 62 vorgesehen sind, für deren Krümmungsradius die gleiche Bedingung wie für den jeweiligen Krümmungsradius in den Eckbereichen des Schaftes gilt. Wie man in Figur 5 erkennt, setzt sich der gekrümmte Abschnitt 62 aus zwei sich schneidenden Kreisbogenabschnitten zusammen, wobei die dadurch entstehende Spitze in der Kontur aber zum Innenraum der Querschnittskontur hin gerichtet ist, so daß die komplementäre Form im Knochen problemlos mit einem rotierenden Fräser gefräst werden kann. Durch diese zusätzlichen Ausbuchtungen und gekrümmten Abschnitte soll die Kontaktoberfläche zwischen dem Schaft und der Wand der Aussparung vergrößert und damit die Rotationsstabilität des Schaftes in dem Knochen erhöht werden.

Die vorstehenden Ausführungsbeispiele zeigen, daß die dem jeweiligen Schaftquerschnitt entsprechende Aussparung mit Hilfe eines Fräskopfes so ausgefräst werden kann, daß die Schaftoberfläche in allen Bereichen an der die Aussparung umgebenden Knochenwand anliegen kann. Dadurch wird ein sofortiger guter Sitz des Schaftes in dem Knochen erreicht und das Einwachsen der Prothese in den Knochen begünstigt.

## Patentansprüche

1. Endoprothese mit einem Verankerungsschaft (12), der zum Einsetzen in eine in einem Röhrenknochen (20) erzeugte Aussparung (18) bestimmt ist, wobei der Verankerungsschaft (12) auf mindestens einem Teil seiner Länge einen mindestens annähernd polygonalen Querschnitt hat, dadurch **gekennzeichnet**, daß sich die Querschnittskonturlinie des Schaftes (12) aus geraden und gekrümmten Linienabschnitten (22, 24, 26, 28 bzw. 38, 40, 42, 44, 60, 62) zusammensetzt, und daß der minimale Krümmungsradius (54) der gekrümmten Linienabschnitte (38, 40, 42, 44, 60, 62) stets größer oder gleich dem Radius eines für die Herstellung der Aussparung (18) im Knochen (20) verwendeten Fräskopfes ist.

2. Endoprothese nach Anspruch 1, dadurch **gekennzeichnet,** daß der Querschnitt des Schaftes (12) auf zumindest einem Teil von dessen Länge im wesentlichen rechteckig ist und daß die Krümmungsmittelpunkte (46, 48) der gekrümmten Linienabschnitte (38, 44) in den Eckbereichen (30, 36) auf der jeweiligen Winkelhalbierenden (50, 52) zweier aneinandergrenzender Seitenlinien (22, 28 bzw. 26, 28) liegen.

3. Endoprothese nach Anspruch 1 oder 2, dadurch **gekennzeichnet,** daß mindestens ein gekrümmter Linienabschnitt aus Teilabschnitten mit unterschiedlichem Krümmungsradius besteht.

4. Endoprothese nach einem der Ansprüche 1 bis 3, dadurch **gekennzeichnet,** daß mindestens ein gekrümmter Linienabschnitt aus gekrümmten Teilabschnitten besteht, deren Krümmungsmittelpunkte einen Abstand voneinander haben.

5. Endoprothese nach einem der Ansprüche 1 bis 4, dadurch **gekennzeichnet,** daß die Abstände des Krümmungsmittelpunktes (56, 58) eines gekrümmten Linienabschnittes (40, 42) von den durch diesen zu verbindenden Seitenlinien (22, 24 bzw. 24, 26) verschieden sind.

6. Endoprothese nach einem der Ansprüche 1 bis 5, dadurch **gekennzeichnet,** daß im Bereich mindestens einer Seitenlinie (22, 26) ein zusätzlicher gekrümmter Linienabschnitt (60,62) vorgesehen ist, dessen Krümmungsradius nicht kleiner als der minimale Krümmungsradius ist.
